# EUROPÄISCHE PATENTSCHRIFT

(11) **EP 4 027 868 B1**
(45) Veröffentlichungstag und Bekanntmachung des Hinweises auf die Patenterteilung: **07.02.2024**
(21) Anmeldenummer: 20816904.5
(22) Anmeldetag: 14.09.2020
(51) Int. Cl.: A61B 5/00, A61B 5/388, A61N 1/00

(54) **MEDIZINISCHE BZW. CHIRURGISCHE GERÄTSCHAFT**
MEDICAL OR SURGICAL EQUIPMENT
APPAREILLAGE MÉDICAL OU CHIRURGICAL

(30) Priorität: 13.09.2019 DE 102019124719
(43) Veröffentlichungstag der Anmeldung: 20.07.2022
(73) Patentinhaber: Lamadé, Wolfram, 65812 Bad Soden (DE)
(72) Erfinder: Lamadé, Wolfram, 65812 Bad Soden (DE)
(74) Vertreter: Patentanwälte und Rechtsanwalt Weiß, Arat & Partner mbB
(86) Internationale Anmeldenummer: PCT/EP2020/075614
(87) Internationale Veröffentlichungsnummer: WO 2021/048435

(56) Entgegenhaltungen:
- WO-A1-2021/005382
- DE-A1-102007 036 862
- US-A1- 2003 040 785
- KONISHI S ET AL: "Cuff actuator for adaptive holding condition around nerves", SENSORS AND ACTUATORS B: CHEMICAL, ELSEVIER BV, NL, Bd. 83, Nr. 1-3, 15. März 2002 (2002-03-15), Seiten 60-66, XP004344486, ISSN: 0925-4005, DOI: 10.1016/S0925-4005(01)01029-2
- TAKEUCHI S ET AL: "A three-dimensional shape memory alloy microelectrode with clipping structure for insect neural recording", JOURNAL OF MICROELECTROMECHANICAL SYSTEMS, IEEE SERVICE CENTER, US, Bd. 9, Nr. 1, 1. März 2000 (2000-03-01), Seiten 24-31, XP011450813, ISSN: 1057-7157, DOI: 10.1109/84.825773
- CRAMPON M-A ET AL: "New nerve cuff electrode based on a shape memory alloy armature", ENGINEERING IN MEDICINE AND BIOLOGY SOCIETY,1998. PROCEEDINGS OF THE20TH ANNUAL INTERNATIONAL CONFERENCE OF THE IEEE, IEEE - PISCATAWAY, NJ, US, Bd. 5, 29. Oktober 1998 (1998-10-29), Seiten 2556-2559, XP010320413, DOI: 10.1109/IEMBS.1998.744973 ISBN: 978-0-7803-5164-6
- Shimizu Koichi ET AL: "Nerve lnterfaces Micro Electrodes and Adaptive Holding Around Nerves for Selective Stimulating and Recording", Proceedingsof the 2007 JSME Conference on Robotics and Mechatronics, 10. Mai 2007 (2007-05-10), Seiten 1-2, XP055770589, Akita, Japan Gefunden im Internet: URL:https://www.jstage.jst.go.jp/article/j smermd/2007/0/2007__1P1-N05_1/_pdf/-char/j a [gefunden am 2021-02-01]
- JUNGMIN SEO ET AL: "Nerve cuff electrode using embedded magnets and its application to hypoglossal nerve stimulation", JOURNAL OF NEURAL ENGINEERING, INSTITUTE OF PHYSICS PUBLISHING, BRISTOL, GB, Bd. 13, Nr. 6, 20. Oktober 2016 (2016-10-20), Seite 66014, XP020311191, ISSN: 1741-2552, DOI: 10.1088/1741-2560/13/6/066014 [gefunden am 2016-10-20]

## Beschreibung

Die Erfindung betrifft eine medizinische bzw. chirurgische Gerätschaft entsprechend dem Oberbegriff von Anspruch 1

### Stand der Technik

In vielen Fällen ist es bei medizinischen bzw. chirurgischen Eingriffen in den menschlichen oder tierischen Körper wünschenswert, wenn bestimmte Teile des menschlichen oder tierischen Körpers überwacht, stimuliert oder auf andere Weise behandelt werden. Vor allem handelt es sich dabei um Fasern, welche Signale abgeben bzw. empfangen können. Auf all diese Teile des menschlichen oder tierischen Körpers bezieht sich die vorliegende Erfindung, insbesondere aber auf Nerven. Seit Jahren wird hier ein sogenanntes Neuromonitoring durchgeführt, um z.B. zu ermitteln, ob im Operationsgebiet liegende Nervenstrukturen verletzt sind. Andererseits ist es aber auch bekannt, dass Nerven durch elektrische Impulse stimuliert werden können, deren Reaktion wiederum ermittelt und analysiert wird. Die vorliegende Erfindung ist vielfältig einsetzbar.

Aus der US 2007/0043411 A1 ist beispielsweise eine Elektrode zum Anlegen an den Nervus Vagus bekannt. Hierzu weist sie von vorneherein eine bestimmte Krümmung auf, an die ein flexibler Fortsatz anschliesst. Durch die vorher bestimmte Krümmung ist die Kontaktfläche mit dem Nerv sehr begrenzt.

Aus der DE 20 2010 006 586 U1 ist wiederum eine Manschettenelektrode mit Sicherheitsverankerung bekannt, die sich in der Praxis sehr bewährt hat. Allerdings ist das Anlegen dieser Elektrode aufwendig und erfordert die Hand des Operateurs. Ferner ist auch hier die Kontaktfläche zwischen Elektrode und Nerv begrenzt.

Die DE 10 2007036862 A1 zeigt eine Elektrode zur intraoperativen Nervenstimulation mit einem um den zu stimulierenden Nerv herum schlingbaren, zu einer geschlossenen Schlinge schliessbaren Kontaktstreifen aus elastischem, biokompatiblen Material, der mindestens eine dem Nerv zugewandte elektrische leitfähige Kontaktfläche aufweist, die mittels eines Signalübertragungsmittels mit einem Signalgeber oder einem Signalaufnehmer verbindbar ist.

Die US 2003/0040785 A1 befasst sich ebenfalls mit einer Elektrode mit einem Trägerelement, welches dünn und flexibel ausgebildet ist und um einen Nerv gelegt werden kann.

In JUNGMIN SEO ET AL: "Nerve cuff electrode using embedded magnets and its application to hypoglossal nerve stimulation", JOURNAL OF NEURAL ENGINEERING, INSTITUTE OF PHYSICS PUBLISHING; BRISTOL, GB, . 13, Nr. 6, 20. Oktober 2016 (2016-10-20), Seite 66014, XP020311191, ISSN: 1741 - 2552, DOI: 10.1088/1741-2560/13/6066014 (gefunden am 2016-10-20) wird eine Elektrode für Nerven gezeigt, bei der in das Trägermaterial Magnete eingebettet sind. Durch diese Magnete erfolgt eine Halterung.

Die Veröffentlichungen TAKEUCHI S ET AL: "A three-dimensional shape memory alloy microelectrode with clipping structure for insect neural recording". JOURNAL OF MICROELEKTROOMECHANICAL SYSTEMS, IEEE SERVICE CENTER, US, Bd 9, Nr. 1, 1. März 2000 (2000-03-01), Seiten 24-32, XP011450813, ISSN: 1057-7157, DOI: 10.1109/84.825773 und CRAMPON M-A ET AL: "New nerve cuff electrode based on a shape memory alloy armature", ENGINEERING IN MEDICINE AND BIOLOGY SOCIETY, 1998. PROCEEDINGS OF THE 20TH ANNUAL INTERNATIONAL CONFERENCE OF THE IEEE, IEEE - PISCATAWAY, NJ, US, Bd 5, 29. Oktober 1998 (1998-10-29), Seiten 2556-2559, XP010320413, DOI: 10.1109/IEMBS.1998.744973, ISBN: 978-0-7803-5164-6 befassen sich mit dem Anlegen von Mikroelektroden an Nerven, wobei die Mikroelektrode eine Formänderung durch den Erinnerungseffekt der entsprechenden Metalllegierung erfährt.

Aus KONISHI S ET AL: "Cuff actuator for adaptive holding condiction around nerves", SENSORS AND ACTUATORS B: CHEMICAL, ELSEVIER BV, NL, Bd.83, Nr. 1-13, 15. März 2022 (2002-03-15), Seiten 60-66, XP004344486, ISSN: 0925-4005, DOI: 10.1016/S0925-4005(01)01029-2 und ähnlich auch aus Shimizu Koichi ET AL: "Nerve Interfaces Micro Electrodes and Adaptive Holding Around Nerves for Selective Stimulating and Recording", Proceedings of the 2007 JSME Conference on Robotics and Mechatronics, 10. Mai 2007 (2007-05-10), Seiten 1-2, XP055770589, Akita, Japan, Gefunden im Internet: U RL:httpa:1 Iwww.jstage.jst.go.JP/article/jsmermd/2007 10/2007 _1 P1 N05_1 I_pd f/-char/ja (gefunden am 2021-02-01) ist eine medizinische Gerätschaft, der im Oberbegriff von Anspruch 1 genannten Art bekannt, welche aus einem SMA-Aktuator und einem Bereich mit pneumatischen Ballons besteht. Der SMA-Aktuator dient dazu, die ursprüngliche Form der Elektrode einzuhalten. Die pneumatischen Ballons tragen zu einer Feinjustierung bei. Der Druck eines individualen Ballons kann unabhängig kontrolliert werden, um gewünschte Haltebedingungen zu erzielen. Aus diesem Grunde sind die pneumatischen Ballons auch quer zur Längsrichtung des Manschettenaktuators angeordnet.

### Aufgabe

Aufgabe der vorliegenden Erfindung ist es, eine Gerätschaft der oben genannten Art zu schaffen, die der Operateur leicht anlegen kann, die eine grosse Kontaktfläche mit dem organischen Signalsender bzw. Signalempfänger aufweist und die wiederum auch leicht zu entfernen ist.

### Lösung der Aufgabe

Zur Lösung der Aufgabe führen die Merkmale des kennzeichnenden Teils von Anspruch 1

Im vorliegenden Fall ist daran gedacht, dass sich der Trägerstreifen in Form einer Spirale bzw. einer Helix um den organischen Signalsender bzw.-empfänger herumwindet. Das bedeutet, dass zum Anlegen des Trägerstreifens an den Signalsender bzw.- empfänger die Hand des Operateurs nicht mehr notwendig ist, wodurch jegliche hygienische Einflüsse bzw. Verletzungsgefahren vermieden werden. Der Trägerstreifen wickelt sich von selbst um den Signalsender bzw.- empfänger, ohne dass ist es eines Zutuns des Operateurs bedarf. Ferner ist natürlich von ganz erheblichem Vorteil, dass das Umwickeln bzw. Umwinden des Signalsenders bzw.- empfängers sehr eng erfolgt, sodass ein sehr guter Kontakt zwischen dem Trägerstreifen und dem Signalsender bzw.- empfänger hergestellt wird. Die Übertragung eines Signals wird hierdurch wesentlich verbessert.

Bevorzugt ist der Trägerstreifen sichelförmig oder L-förmig ausgebildet. Das bedeutet, dass er sich beim Anlegen an den Signalsender bzw.- empfänger nicht um sich selbst schlingt, sondern als Helix einen möglichst grossen Kontaktbereich mit dem Signalsender bzw.- empfänger ausbildet. Ferner ist auch daran gedacht, eine vorgeformte helikale Elektrode aus einer geraden Schutzhülle herausgleiten zu lassen, damit sie danach ihre helikale Form annehmen kann. Bekannt ist dies bei den sogenannten Pigtail-Kathetern.

Des weiteren besteht der Trägerstreifen aus einem sehr dünnen, flexiblen Material, quasi aus einer Folie, die sich um den Signalsender bzw.-Empfänger legt. Zum Abziehen genügt es, wenn z.B. an einem Leiter zu dem Trägerstreifen gezogen wird, so dass sich dann dieser Trägerstreifen von dem Signalsender bzw.- empfänger abwickelt.

Erfindungsgemäss ist daran gedacht, dass die Formänderung durch eine hydraulische oder pneumatische Einrichtung erfolgt. Hierzu ist in dem Trägerstreifen ein Kanal für ein hydraulisches oder pneumatisches Medium integriert sein, der so ausgestaltet ist, dass der Trägerstreifen bei Einführung des Mediums eine Formänderung erfährt. Das entsprechende hydraulische oder pneumatische Medium befindet sich in diesem Fall in einem Reservoir, welches ebenfalls in dem Trägerstreifen integriert ist. Wird dieses Reservoir unter Druck gesetzt, wird das hydraulische oder pneumatische Medium in den Kanal gedrückt, der den Trägerstreifen durchzieht. Dadurch wird ein vorher spiralartig aufgerollter Trägerstreifen gerade ausgerichtet. Wird der Druck auf das Reservoir aufgehoben, strömt das Medium wieder in das Reservoir zurück, und der Trägerstreifen kehrt in seine ursprüngliche, spiral- oder helixartige Form zurück. Dies kann natürlich noch dadurch begünstigt werden, dass der Trägerstreifen aus einem Material mit Memory-Effekt besteht.

### Figurenbeschreibung

Weitere Vorteile, Merkmale und Einzelheiten ergeben sich aus der nachfolgenden Beschreibung bevorzugter Ausführungsbeispiele sowie anhand der Zeichnung; diese zeigt in
**Figur** 1 eine Draufsicht auf eine medizinische bzw. chirurgische Gerätschaft ausserhalb Gebrauchslage;
**Figur 2** eine perspektivische Ansicht der medizinischen bzw. chirurgischen Gerätschaft in Gebrauchslage;
**Figur 3** eine perspektivische Ansicht auf einer erfindungsgemässen medizinischen bzw. chirurgischen Gerätschaft ausserhalb Gebrauchslage;
**Figur 4** eine perspektivische Ansicht der medizinischen bzw. chirurgischen Gerätschaft gemäss Figur 3 entlang Linie IV - IV;

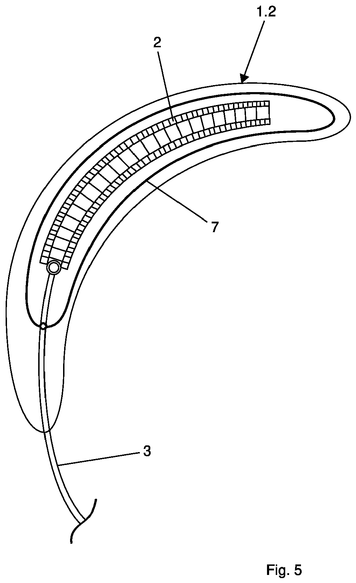

**Figur 5** eine perspektivische Ansicht auf ein weiteres Ausführungsbeispiel der erfindungsgemässen medizinischen bzw. chirurgischen Gerätschaft ausserhalb Gebrauchslage.

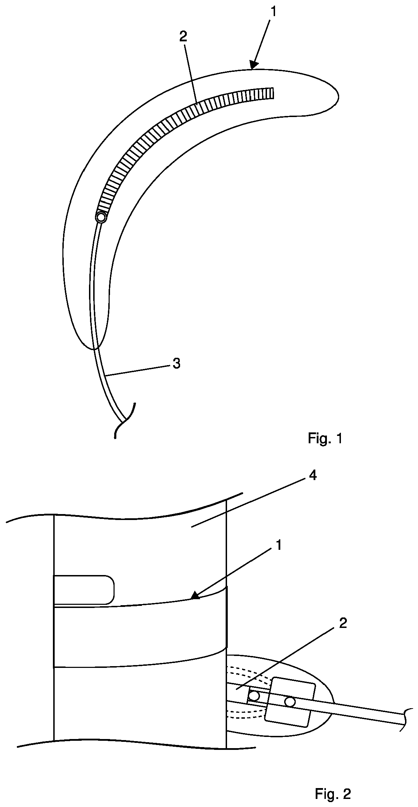

Gemäss Figur 1 weist eine erfindungsgemässe medizinische bzw. chirurgische Gerätschaft einen Trägerstreifen 1 auf, der mit einem Signalüberträger 2 belegt ist. In der Regel handelt es sich bei dem Signalüberträger 2 um eine Elektrode. An den Signalüberträger 2 schliesst eine Leiter 3 zu einer beliebigen elektrischen Gerätschaft an, mit der ein Impuls des Signalüberträgers empfangen bzw. ein Impuls an den Signalüberträger 2 ausgegeben werden kann.

Die Funktionsweise der vorliegenden Erfindung ist folgende:
Wesentlich für die vorliegende Erfindung ist die Ausgestaltung des Trägerstreifens 1. Dieser soll sich, wie in Figur 2 gezeigt, in seiner Form verändern können und zwar in der Weise, dass er sich beispielsweise um einen Nerv 4 als Beispiel eines organischen Signalsenders bzw.- empfängers legen kann. Damit erhält der Signalüberträger 2 einen quasi flächigen Kontakt mit dem Signalsender bzw.- empfänger 4. Die bevorzugt gewählte L-Form (oder auch Sichel-, Bananen- oder Bumerangform) des Trägerstreifens 1 ermöglicht dabei auch eine helixartige Umschlingung des Signalsenders bzw.- empfängers 4, wodurch die Kontaktmöglichkeit nochmals vergrössert wird.

Allerdings besteht der Trägerstreifen 1 aus einem derart flexiblen Material, dass die Formänderung durch leichtem Zug an dem Leiter 3 überwunden und der Trägerstreifen von dem Nerv 4 abgezogen werden kann.

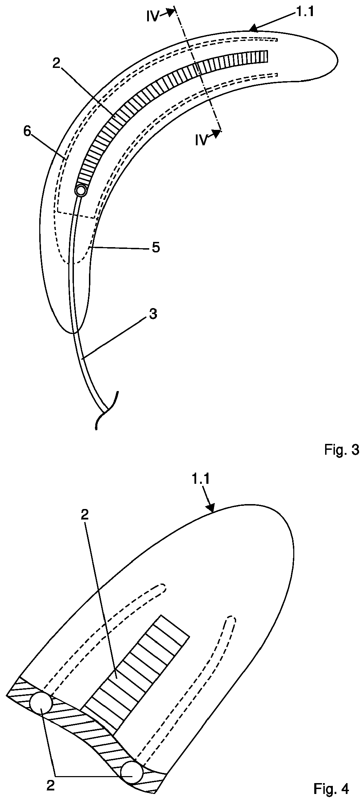

Erfindungsgemäss soll gemäss den Figuren 3 und 4 ist in einem Trägerstreifen 1.1 eine hydraulische bzw. pneumatische Einrichtung integriert. Diese besteht aus einem Reservoir 5 und zumindest einer, bevorzugt zwei Leitungen 6.1 und 6.2 für ein hydraulisches bzw. pneumatisches Medium. Diese verläuft entlang dem Trägerstreifen 1.1 und dem Signalüberträger 2. Bei Druck auf das Reservoir 5 strömt ein hydraulisches bzw. pneumatisches Medium in die Leitung 6.1, 6.2 und bewirkt, dass sich der Trägerstreifen 1.1 gerade und flach ausrichtet. Wird der Druck auf das Reservoir 5 aufgehoben, strömt das hydraulische bzw. pneumatische Medium wieder zurück in das Reservoir 5 und der Trägerstreifen 1.1 kehrt zu seiner ursprünglichen helixartig gewundenen Form zurück. Dies kann beispielsweise dadurch unterstützt werden, dass der Trägerstreifen 1.1 aus einem Material mit Memory-Effekt besteht.

Wird somit die erfindungsgemässe Gerätschaft während einer Operation beispielsweise an einem Nerv 4 benötigt, kann der Operateur, beispielsweise mit einer Pinzette, auf das Reservoir 5 drücken, wodurch das hydraulische bzw. pneumatische Medium in die Leitung 6.1, 6.2 einströmt und den Trägerstreifen 1.1 gerade ausrichtet. Jetzt kann der Operateur den Trägerstreifen 1.1 an den Nerv 4 anlegen, den Druck auf das Reservoir 5 aufheben und der Trägerstreifen 1 schlingt sich um den Nerv 4.

Beim Abnehmen der Gerätschaft von dem Nerv 4 genügt es in der Regel, wenn der Trägerstreifen 1.1 so flexibel ausgebildet wird, dass er sich bei Zug an dem Leiter 3 von dem Nerv 4 abrollt. Sollte dies nicht ausreichen, kann der Operateur wiederum Druck auf das Reservoir 5 ausüben, sodass sich der Trägerstreifen 1.1 gerade stellt.

### Bezugszeichenliste

| | | | | | |
|---|---|---|---|---|---|
| 1 | Trägerstreifen | 34 | | 67 | |
| 2 | Elektroden | 35 | | 68 | |
| 3 | Leiter | 36 | | 69 | |
| 4 | Nerv | 37 | | 70 | |
| 5 | Reservoir | 38 | | 71 | |
| 6 | Leitung | 39 | | 72 | |
| 7 | Widerstandsdraht | 40 | | 73 | |
| 8 | | 41 | | 74 | |
| 9 | | 42 | | 75 | |
| 10 | | 43 | | 76 | |
| 11 | | 44 | | 77 | |
| 12 | | 45 | | 78 | |
| 13 | | 46 | | 79 | |
| 14 | | 47 | | | |
| 15 | | 48 | | | |
| 16 | | 49 | | | |
| 17 | | 50 | | | |
| 18 | | 51 | | | |
| 19 | | 52 | | | |
| 20 | | 53 | | | |
| 21 | | 54 | | | |
| 22 | | 55 | | | |
| 23 | | 56 | | | |
| 24 | | 57 | | | |
| 25 | | 58 | | | |
| 26 | | 59 | | | |
| 27 | | 60 | | | |
| 28 | | 61 | | | |
| 29 | | 62 | | | |
| 30 | | 63 | | | |
| 31 | | 64 | | | |
| 32 | | 65 | | | |
| 33 | | 66 | | | |

## Patentansprüche

1. Medizinische bzw. chirurgische Gerätschaft zum Empfangen von Signalen bzw. zum Ausgeben von Signalen von bzw. an organische Signalsender bzw.- empfänger (4), wie insbesondere Nerven, wobei auf oder in zumindest einem Trägerstreifen (1.1) zumindest ein Signalüberträger (2) für Signale von bzw. Signale auf den organischen Signalsender bzw. - empfänger (4) vorgesehen ist, der mit dem organischen Signalsender bzw.- empfänger (4) in Kontakt bringbar ist, wobei sich der Trägerstreifen (1.1) in Form einer Spirale bzw. einer Helix um den organischen Signalsender bzw.- empfänger (4) herumwindet und der Trägerstreifen (1, 1.1, 1.2) sich bei Änderung eines in ihm vorhandenen Mediums oder durch ein in ihn einführbares Medium seine Form so ändert, dass sie sich dem organischen Signalsender bzw.- empfänger (4) anpasst, und wobei in dem Trägerstreifen (1.1) eine hydraulische bzw. pneumatische Einrichtung integriert ist,
**dadurch gekennzeichnet,**
**dass** die hydraulische bzw. pneumatische Einrichtung ein gerades und flaches Ausrichten des Trägerstreifens (1.1) bewirkt und aus einem Reservoir (5) und zumindest einer Leitung (6.1,6.2) für ein hydraulisches bzw. pneumatisches Medium besteht, die entlang dem Trägerstreifen (1.1) und dem Signalüberträger (2) verläuft, wobei bei Druck auf das Reservoir (5) das hydraulische bzw. pneumatische Medium in die Leitung (6.1,6.2) strömt und bewirkt, dass sich der Trägerstreifen (1.1) gerade und flach ausrichtet, während bei Aufhebung des Drucks auf das Reservoir (5) das hydraulische bzw. pneumatische Medium wieder in das Reservoir (5) zurückströmt und der Trägerstreifen (1.1) zu seiner ursprünglichen helixartig gewundenen Form zurückkehrt.

## Claims

1. Medical or surgical equipment for receiving signals or for outputting signals from or to organic signal transmitters or receivers (4), such as in particular nerves, wherein on or in at least one carrier strip (1.1) at least one signal carrier (2) for signals from or signals to the organic signal transmitter or receiver (4) is provided, which can be brought into contact with the organic signal transmitter or receiver (4), wherein the carrier strip (1.1) winds in the form of a spiral or a helix around the organic signal transmitter or receiver (4) and the carrier strip (1, 1.1, 1.2) changes its shape when there is a change in a medium surrounding it or in a medium present in it or by a medium which can be introduced into it, in such a way that it adapts to the organic signal transmitter or receiver (4), and wherein a hydraulic or pneumatic device is integrated in the carrier strip (1.1),
**characterized in that**
the hydraulic or pneumatic device effects a straight and flat alignment of the carrier strip (1.1) and consists of a reservoir (5) and at least one conduit (6.1, 6.2) for a hydraulic or pneumatic medium which runs along the carrier strip (1.1) and the signal transmitter (2), wherein when pressure is applied to the reservoir (5), the hydraulic or pneumatic medium flows into the conduit (6.1, 6.2) and causes the carrier strip (1.1) to align itself straight and flat, while when the pressure on the reservoir (5) is released, the hydraulic or pneumatic medium flows back into the reservoir (5) and the carrier strip (1.1) returns to its original helically wound shape.

## Revendications

1. Équipement médical ou chirurgical destiné à recevoir des signaux ou à transmettre des signaux provenant d'un ou à un émetteur ou récepteur organique de signaux (4), comme en particulier des nerfs, dans lequel il est prévu, sur ou dans au moins une bande porteuse (1.1), au moins un transmetteur de signaux (2) pour des signaux provenant du ou des signaux sur l'émetteur ou le récepteur organique de signaux (4), lequel transmetteur de signaux peut être mis en contact avec l'émetteur ou le récepteur organique de signaux (4), dans lequel la bande porteuse (1.1) est bobinée autour de l'émetteur ou du récepteur organique de signaux (4) sous la forme d'une spirale ou d'une hélice et la bande porteuse (1, 1.1, 1.2) change de forme lors du changement d'un fluide présent dans celle-ci ou sous l'action d'un fluide qui peut y être introduit, de telle sorte que cette forme se conforme à l'émetteur ou le récepteur organique de signaux (4), et dans lequel un dispositif hydraulique ou pneumatique est intégré dans la bande porteuse (1.1),
**caractérisé en ce**
**que** le dispositif hydraulique ou pneumatique entraîne un alignement droit et plat de la bande porteuse (1.1) et est constitué d'un réservoir (5) et d'au moins une conduite (6.1, 6.2) pour un fluide hydraulique ou pneumatique, lequel dispositif s'étend le long de la bande porteuse (1.1) et du transmetteur de signaux (2), dans lequel, en cas de pression sur le réservoir (5), le fluide hydraulique ou pneumatique s'écoule dans la conduite (6.1, 6.2) et entraîne l'alignement droit et plat de la bande porteuse (1.1) tandis que, en cas de suppression de la pression exercée sur le réservoir (5), le fluide hydraulique ou pneumatique est refoulé dans le réservoir (5) et la bande porteuse (1.1) revient à sa forme d'origine bobinée en hélice.
